# EUROPEAN PATENT APPLICATION

(11) **EP 4 360 553 A1**
(43) Date of publication of application: **01.05.2024**
(21) Application number: 22204485.1
(22) Date of filing: 28.10.2022
(51) Int. Cl.: A61B 5/16, A61B 5/00

(54) **ASSESSING COGNITIVE PERFORMANCE OF A SUBJECT**

(71) Applicant: Koninklijke Philips N.V., 5656 AG Eindhoven (NL)
(72) Inventor: LEUFKENS, Timmy Robertus Maria, 5656AG Eindhoven (NL); VAN EE, Raymond, 5656AG Eindhoven (NL); DENISSEN, Adrianus Johannes Maria, 5656AG Eindhoven (NL); VAN DOOREN, Marieke, 5656AG Eindhoven (NL); SPELT, Hanne Adriana Alijda, 5656AG Eindhoven (NL)
(74) Representative: Philips Intellectual Property & Standards

(57) **Abstract**

Proposed concepts thus aim to provide schemes, solutions, concepts, designs, methods and systems pertaining to aiding and/or improving cognitive assessment of a subject. In particular, an alertness value describing an alertness level of the subject while executing cognitive task is determined. Proposed embodiments determine the alertness level by analysis of biological cycle data of the subject, which may provide valuable information in understanding the subject's alertness. Thus, a modification to the performance value of the subject in executing the cognitive task is determined. In this way, proposed embodiments may facilitate improved (i.e. more accurate) cognitive assessment of a subject.

## Description

### FIELD OF THE INVENTION

The present invention relates to the field of cognitive assessment, and in particular to the field of assessing cognitive performance of a subject (i.e. an individual or patient).

### BACKGROUND OF THE INVENTION

In the field of cognitive assessment, specialized tests are used to assess the cognitive health of subjects. For example, a subject is instructed to undertake a task that is carefully designed to exercise certain cognitive functions. The subject's performance on the task provides insights that a professional (e.g., a medical professional) can use to assess the subject's cognitive health. Examples of cognitive capabilities that are commonly assessed are memory, learning, inductive reasoning, and decision making. Examples of cognitive tests include the clock drawing test, the Montreal Cognitive Assessment (MoCA), the Mini-Mental State Exam (MMSE), and the Mini-Cog.

The alertness (i.e. a sleepiness, drowsiness, arousal, etc.) level of an individual has been shown to affect cognitive functioning while performing a wide array of cognitive tasks. Sleepy subjects perform particularly badly at tasks that involve attention and working memory, as well as tasks that involve long-term memory and decision making. Further, alertness has an even greater impact on cognitive performance for subjects with brain disorders, such as traumatic head injury, stroke, dementia, developmental disorders, and psychiatric disorders.

Moreover, an alertness level of a subject is impacted by a wide variety of factors. The impact of the subject's biological cycles can be particularly indicative of a subject's alertness, as well as a quality of sleep, and a time of day. Moreover, internal and external circumstances may make a subject more or less alert.

Thus, there is a need to optimize the circumstances for a subject to take a cognitive assessment and/or to improve accuracy of a cognitive assessment in view of factors that may affect a subject's alertness.

### SUMMARY OF THE INVENTION

The invention is defined by the claims.

According to examples in accordance with an aspect of the invention, there is provided a method for assessing cognitive performance of a subject, the method comprising:
obtaining a performance value describing the subject's performance in executing a cognitive task for cognitive assessment of the subject;
obtaining biological cycle data describing parameters of at least one biological cycle of the subject;
analyzing the biological cycle data to determine an alertness value describing an alertness level of the subject during the cognitive task; and
determining a modification to the performance value based on the alertness level.

Proposed concepts thus aim to provide schemes, solutions, concepts, designs, methods and systems pertaining to aiding and/or improving cognitive assessment of a subject. In particular, an alertness value describing an alertness level of the subject while executing cognitive task is determined. Proposed embodiments determine the alertness level by analysis of biological cycle data of the subject, which may provide valuable information in understanding the subject's alertness. Thus, a modification to the performance value of the subject in executing the cognitive task is determined. In this way, proposed embodiments may facilitate improved (i.e. more accurate) cognitive assessment of a subject.

It is proposed that biological cycles/rhythms are indicative of a subject's alertness level. The term biological rhythm/cycle refers to a number of human biological cycles each having a different time frame, such as circadian, diurnal, and menstrual cycles. Such biological cycles vary between subjects, in that they are dependent an internal bodily clock and influenced by different internal (e.g. hormones) and external (e.g. light) factors. Each biological rhythm effects a number of bodily functions, such as metabolism, heart rate, blood pressure, body temperature, hormone levels and urine production, but also cognitive and mental functions, such as mood, alertness and concentration.

Embodiments therefore propose obtaining an insight into the alertness of a subject during performance of a cognitive test by analysis of the biological cycle of the individual subject. Such insight(s) about the subject's alertness level during execution of a cognitive task may then be used to determine an appropriate modification for an obtained performance score in executing the cognitive task. In other words, information about the subject's alertness level may be leveraged to compensate or correct for one or more drops in performance in executing a cognitive task.

Moreover, it is proposed that leveraging parameters of biological cycles/rhythms of the subject enables improved (i.e. more accurate) determination of an alertness level of the subject. Alternative methods for determining an alertness level may be inherently subjective (i.e. questionnaires), or require specialized equipment (i.e. cameras tracking eye movement). Put another way, biological cycle data provides a reliable means for retrospective (or real time) determination of an alertness level of the subject during execution of a cognitive task, as well as projection of a future alertness of the subject.

Furthermore, some embodiments propose utilizing information about the subject's alertness level (based on biological cycle parameters of the subject) in order to optimize/alter the circumstances for a subject to perform a cognitive task for assessment (i.e. perform the task when they are likely to be most alert). In this way, proposed embodiments may facilitate improved (i.e. more accurate) cognitive assessment of a subject.

In some embodiments, the method may further comprise obtaining sleep data indicative of a quality of sleep of the subject prior to executing the cognitive task. In this case, determining the alertness value may be further based on analyzing the sleep data.

The quality of sleep of a subject is a large determining factor of the subject's subsequent alertness levels. Indeed, such data in conjunction with biological cycle data may provide an accurate estimation/prediction of an alertness level of the subject at different times of day, and therefore during execution of a cognitive task.

For example, disrupted sleep can greatly reduce cognitive performance of the subject, and is even more pronounced in individuals with brain disorders such as traumatic head injury, stroke, dementia, developmental disorders, and psychiatric disorders. Therefore, information about a subject's sleep and biological cycles provides useful insights/information as to the subject's alertness. In this way, proposed embodiments may facilitate improved (i.e. more accurate) cognitive assessment of a subject.

Further, analyzing the sleep data may comprise processing the sleep data to determine a homeostatic pressure value describing sleep-pressure affecting the subject during the cognitive task. Thus, determining the alertness value may be further based on analyzing the homeostatic pressure value.

The homeostatic process represents a progressive build-up of sleep pressure across time awake, which dissipates across time asleep. Sleepiness results from high homeostatic pressure for sleep, and therefore is indicative of an alertness of a subject performing a cognitive task.

Indeed, analysis of a homeostatic pressure alongside biological cycle data (i.e. a circadian rhythm) of the subject provides an accurate means for determining an alertness of the subject. A low alertness level of the subject may arise from a high homeostatic pressure for sleep and low circadian pressure for wakefulness. Thus, understanding these factors affecting a subject during execution of a cognitive task may enable a more accurate assessment of the subject's performance.

The sleep data of the subject may comprise a start time of sleep, an end time of sleep, sleep stage data, and a perceived quality of sleep.

Not only does a length of sleep indicate the quality/adequacy of the sleep of the subject (and associated alertness level during the day), but also the stages of sleep experienced by the subject and their perceived quality of sleep. For example, the stages of sleep include light, deep and REM sleep, as well as any wakeful periods during a sleep cycle. Thus, obtaining the above sleep data leads to an improved (i.e. more accurate) assessment of an alertness level of a subject during execution of a cognitive task.

In some embodiments, the method may further comprise determining a cognitive testing recommendation based on the determined alertness value, the cognitive testing recommendation indicating timing of another cognitive task for cognitive assessment of the subject.

As an alternative (or in addition to) determining a modification to the performance value based on the alertness value, it is proposed to generate a recommendation for the cognitive assessment. The recommendation may be for appropriate timing of the cognitive task, being a time when the subject is expected to be at their most alert (based on biological cycle data). This may enable the acquisition of a performance vale that is more reflective of the subject's true ability in executing the task.

Moreover, the performance value may describe the subject's performance in executing a plurality of cognitive tasks, wherein each of the plurality of cognitive tasks have a different task parameter describing a characteristic of the cognitive task. Thus, determining the alertness value may be further based on analyzing a variation in the subject's performance in executing each of the plurality of cognitive tasks.

It is also possible to derive an alertness of the subject by delineating performance (i.e. a response time) in performing different cognitive tasks. For example, more complex cognitive tasks take an increasing amount of time for a subject to perform. However, the rate of increase is smaller for those who are more alert. Thus, by assessing the subject's performance in executing a plurality of cognitive tasks (with different task parameters, and thus varying difficulties), an alertness value of the subject may be determined.

The task parameter (which is different between tasks) may comprise at least one of a stimulus intensity, a signal intensity, a memory load, a stimulus-response compatibility, and a time uncertainty.

Accordingly, an improved determination of alertness is achieved, which may be useful in better understanding the subject's performance.

In exemplary embodiments, the method may further comprise comparing the subject's performance in executing the cognitive task with a reference performance value. Thus, determining the alertness value is further based on analyzing the comparison.

Indeed, the reference performance value may be based on a historic measure of the subject's performance in executing a previous cognitive task.

By way of explanation, the performance of the subject compared to a reference performance may also be indicative of an alertness of the subject. For example, the reference performance may indicate a performance when the subject is alert/at a reference alertness value. Thus, a difference between the subject's performance value and the reference performance value may provide useful information for understanding the alertness level of the subject.

In some embodiments, the biological cycle data of the subject may describe at least one of a circadian cycle, a menstrual cycle, a diurnal cycle, an ultradian cycle, an infradian cycle, and a circannual cycle of the subject.

Each of these biological cycles/rhythms impact various aspects of the subject, and so may directly or indirectly impact an alertness of the subject depending on where along the cycle the subject is. Thus, by taking at least one of these cycles into account, insights into the alertness of the subject may be provided.

In some embodiments, the method may further comprise obtaining at least one of an internal state describing a psychophysical condition of the subject during the cognitive task, and an external circumstance describing surroundings of the subject during the cognitive task. Accordingly, determining the alertness value may be further based on analyzing at least one of the internal state and the external circumstances of the subject.

Specifically, the internal state may comprise at least one of a mood, an emotional state, a physical condition, and a pharmacological substance intake. Also, the external circumstance may comprise at least one of an ambient temperate, an ambient noise level, an ambient light level, and a task constraint.

Indeed, it is well documented how internal factors and external circumstances/factors can also impact an alertness of the subject. For example, a subject having recently consumed coffee, in a good mood and in a cold environment may be more alert than a subject who is hungry, in a distracted mood and being surrounded by people talking. Thus, such factors may be taken into account to improve (i.e. increase an accuracy of) a determination of an alertness level of the subject.

In certain embodiments, the method further comprises providing a cognitive test to the subject comprising the cognitive task.

According to another aspect, there is provided a computer program comprising computer program code means adapted, when said computer program is run on a computer, to implement the method for assessing cognitive performance of a subject according to a proposed embodiment.

According to yet another aspect of the invention, there is provided a system for assessing cognitive performance of a subject, the system comprising:
an interface configured to obtain a performance value describing the subject's performance in executing a cognitive task for cognitive assessment of the subject; and obtain biological cycle data describing parameters of at least one biological cycle of the subject; and
a processor configured to: analyze the biological cycle data to determine an alertness value describing an alertness level of the subject during the cognitive task; and determine a modification to the performance value based on the alertness level.

Thus, there may be proposed concepts for assisting and/or improving cognitive assessment of a subject via the determination/provision of biological cycle data for determination of an alertness of a subject during the performance of a cognitive test. Provision of information about the alertness level of the subject provides additional outcome measures and/or facilitate modification of a cognitive assessment workflow. The proposed concept(s) may thus enable improved (e.g. more accurate) cognitive assessment of a subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figs. 1A and 1B are graphs representing a simplified relationship between a homeostatic pressure and a circadian rhythm of a subject over time;
Fig. 2 is a graph showing a simplified representation of a stimulus quality with response time of a subject with differing alertness levels;
Fig. 3 is a flow diagram of a method for assessing cognitive performance of a subject according to an embodiment of the invention;
Fig. 4 is a simplified block diagram of a system for assessing cognitive performance of a subject according to another embodiment of the invention; and
Fig. 5 is a simplified block diagram of a computer within which one or more parts of an embodiment may be employed.

### DETAILED DESCRIPTION OF THE EMBODIMENTS

The invention will be described with reference to the Figs.

It should be understood that the Figs are merely schematic and are not drawn to scale. It should also be understood that the same reference numerals are used throughout the Figs to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

The invention proposes concepts for improving cognitive assessment of a subject, which may provide the subject and/or a caregiver with greater information surrounding the performance of the subject in executing a cognitive task. In particular, embodiments may provide a method and/or system which determines an alertness level of the subject executing the cognitive task based on biological cycle data of the subject. Indeed, it has been shown that the alertness level of the subject greatly impacts their performance, and that the alertness level is highly dependent on subject-specific biological cycles/rhythms (e.g. a circadian rhythm).

Specifically, proposed concepts may provide an approach to correcting performance values obtained from cognitive assessments/tests in light of biological cycle data of the subject. For example, a subject may perform better at tasks at certain points in various biological cycles than at other points, as biological cycles impact many internal factors of the subject. Accordingly, insights provided by such data can be used to contextualize performance values of the subject, as well as providing the potential for recommendations regarding the timing of such cognitive examinations.

Overall, it is proposed that biological cycle data provides useful information/insights as to the alertness of the subject. As the alertness of the subject may heavily influence a performance of the subject in executing a cognitive task, knowledge of biological cycles is key for accurately understanding performance scores generated by a subject performing a cognitive task, or tasks.

Accurately understanding performance scores relating to cognitive tasks may be particularly useful when diagnosing a subject. Indeed, subjects with neurological conditions may be more heavily impacted by a reduced alertness level. Thus, providing this context, and perhaps adjusting for it, may provide valuable information for properly understanding the state of a subject.

By way of explanation, the term biological cycle/rhythm is often associated with the circadian rhythm (i.e. the human 24-hour rhythm). This is a rhythm resulting from a series of bodily functions regulated by the internal biological clock. The rhythm controls cycles like sleep and wakefulness, body temperature and hormone secretion.

However, there are other human biological rhythms as well. They differ in time frame and are influenced by different internal (e.g. hormones) and external (e.g. light) factors. This includes: the diurnal cycle (night and day), the circadian cycle (24 hour) the ultradian cycle (less than 24 hour), the infradian/circalunar cycle (1 month) and the circannual cycle (1 year).

The above biological rhythms impact a number of bodily functions, such as metabolism, heart rate, blood pressure, body temperature, hormone levels and urine production, but also cognitive and mental functions, such as mood, alertness and concentration.

The clinical definitions of alertness, sleepiness, drowsiness, and arousal are nuanced, particularly in diagnosis and treatment, but are often used interchangeably. One of the most investigated neurobiological processes that impact alertness is the homeostatic and circadian processes.

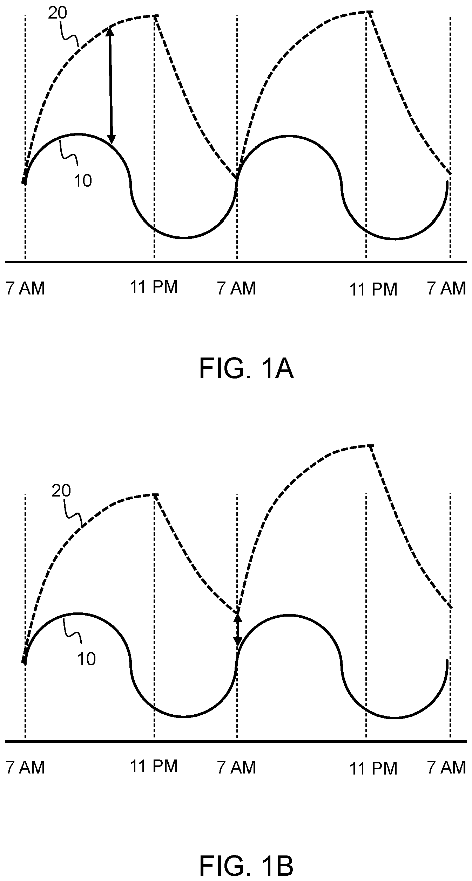

As shown in Fig. 1A, the homeostatic process 20 represents a progressive build-up of sleep pressure (represented by the growing arrow with time during a wake period) across time awake, which dissipates (i.e. narrows) across time asleep. The circadian process 10 (which drives 24 hour rhythms in the brain and body), represents a waxing and waning of wake pressure across time of day. It has been shown that sleepiness (i.e. a lack of alertness) results from high homeostatic pressure for sleep and low circadian pressure for wakefulness, as represented by the arrow in Fig. 1A. Indeed, this trend generally increases as the day progress, until the person sleeps.

Aside from the homeostatic and circadian processes, there are many additional factors that impact alertness, including internal states and external circumstances. Internal states include variables such as psychological states (e.g., mood, anxiety) and pharmacological substances (e.g., caffeine, prescription drugs). External circumstances include variables such as environmental conditions (e.g., ambient temperature, light level) and cognitive task constraints (e.g., task load, time pressure). Alertness levels are determined by the net effect of these concurrent, frequently competing, influences in relation to the homeostatic and circadian processes.

Disrupted sleep, the time of day and an individual's biological rhythms/cycles (e.g. the circadian rhythm) have been shown to impact cognitive functioning in a wide array of cognitive domains. Sleep deprived people (i.e. those with low alertness levels) perform particularly badly in task domains such as attention and working memory, but also on task domain like long-term memory and decision making.

Indeed, the impact of this is shown by the arrow in Fig. 1B, in which disrupted sleep inherently leads to a sleep pressure even after a sleep. The arrow indicates that disrupted sleep leads to an initial sleep-pressure when a person wakes up, which increases the overall sleep-pressure the person experiences during the day.

With respect to circadian rhythms, it has been reported that normal performance levels of subject's performing cognitive tasks can usually be obtained from healthy adults between 10:00 and 14:00 and between 16:00 and 22:00. Yet, outside those hours, attention, working memory and executive function appear to be working below norm.

It should be noted that the impact of disrupted sleep and circadian rhythm on cognitive performance is even more pronounced in individuals with brain disorders such as traumatic head injury, stroke, dementia, developmental disorders, and psychiatric disorders. Therefore, it has been realized that gathering information about a subject's biological cycles (i.e. circadian rhythm) and recent sleep history may be useful when assessing their cognitive performance in a neuropsychological/cognitive examination.

The neurobiology underlying alertness is responsible for measurable changes in the brain and body, such as specific electroencephalographic (EEG) brainwave patterns, pupil diameter, eyelid closure, and the appearance of slow eye movements (SEMs), which can be distinguished in response to changes in the homeostatic and circadian processes.

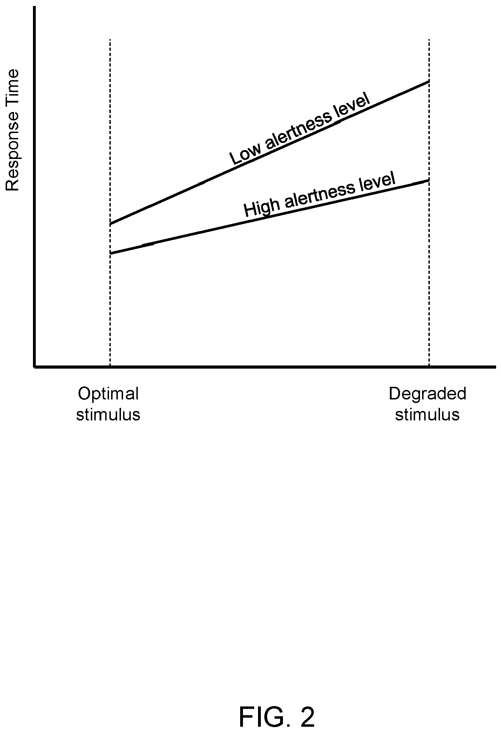

However, as shown in Fig. 2, one can also derive levels of sleepiness or arousal by delineating the response time in a cognitive task. Manipulation of experimental variables (such as the quality of a stimulus) also presents a means for assessing the effort a subject is putting into the performance during a cognitive assessment.

By way of illustrative example, imagine a response task in which a subject's response time is 300 milliseconds. If it is a simple response task, with optimal stimulus intensity (e.g. bright or loud enough) and signal quality (e.g. no background noise), with low memory load (e.g. no extra task to memorize another stimulus), with optimal stimulus-response compatibility (e.g. stimulus appearing on right side of screen should be responded to with right hand push on button), and no time uncertainty (e.g. predictable appearance of stimulus), then it could be derived that that is the base level of the patient's effort. This is illustrated by the response time during the optimal stimulus in Fig. 2.

A manipulation of one of the above experimental variables will typically lead to an increase of response time, as represented by the graph of Fig. 2. A subject at a low alertness level will have a greater difference in response time in performing the task with different stimulus qualities than the subject at a high alertness level

Using the above knowledge, embodiments of the present invention propose overcoming one or more of the following problems:
(i) Unreliable cognitive assessment when the subject is assessed at a time outside an optimal human biological cycle time window (e.g. outside optimal circadian time window);
(ii) Unreliable cognitive assessment when sleep is disrupted. This problem is compounded when knowledge about sleep is lacking;
(ii) Incorrect/inaccurate interpretation of cognitive assessment results when knowledge of sleep, human biological cycle are not known; and
(iv) Incorrect/inaccurate interpretation of cognitive assessment when effect of level of sleepiness (due to biological cycles, disrupted sleep, etc.) is misunderstood/ignored.

Indeed, to overcome the above problem embodiments of the invention may provide the following main elements:
(a) A user interface to present a cognitive test/exam prompting the subject to perform/execute at least one cognitive task;
(b) A means to capture subject biological cycle data (e.g. by a chronotype questionnaire for circadian rhythm, menstrual phase for circalunar rhythm), and potentially sleep data (e.g. a sleep wearable);
(c) A processing unit (having an algorithm/method/code) for:
   (i) collecting the cognitive test stimuli (i.e. parameters/characteristics of the cognitive task(s)), and the cognitive test responses (i.e. a performance value, as well as the sleep data and the biological cycle data;
   (ii) estimating/predicting/determining a level of awareness/sleepiness/arousal based on the sleep and biological data; and
   (iii) correct/modify the performance value and/or provide an examination recommendation including an optimal or improved time to conduct the cognitive examination.

Accordingly, the invention provides additional information and insights that may inform clinicians' diagnosis of cognitive impairment in people with subtle cognitive impairment (e.g. mTBI, MS, stroke). As a result, the invention may facilitate improved subject diagnosis and outcomes.

By way of example, recommendations for (re)scheduling the cognitive (i.e. neurological) assessment sessions, according to biological cycles/rhythms that impact cognitive performance, could be provided by the invention. Indeed, the following are illustrative examples where this may be effective.

The subject's chronotype may be determined (e.g. by means of a chronotype questionnaire, or determining mid-sleep, melatonin saliva test or core body temperature). That is, it is determined whether a patient is a morning or an evening person. The recommendation may thus be to schedule the cognitive examination sessions in the morning for morning-type patients and in the afternoon for evening-type patients. This may mean that the resultant performance value for the subject at these times may be more accurate, and require less (or in some cases, no) modification/adjustment/correction.

A subject's sleep-wake cycle may be determined/assessed. Thus, the cognitive assessment may be scheduled around 3 hours after their regular wake-up time. In a more specific embodiment, a full circadian variation of the core body temperature of the subject may be determined. The assessment recommendation may then be to schedule the cognitive assessment during periods of little variation (indicating alertness).

In a further embodiment, the quality of sleep of the subject prior to (i.e. the night before) the cognitive assessment may be determined. The cognitive examination may thus be rescheduled should the subject not sleep well, to the point that a performance value may greatly differ from a potential performance value, such that a modification to the performance value in light of sleep data may be inaccurate.

Furthermore, data related to long term and short term experienced variations in mood of the subject may be acquired. As explained above, variations in mood are shown to impact cognitive performance as well. The fluctuations in mood can be either be used to adjust the cognitive performance of the patient (e.g. a lower/subdued mood results in a lower cognitive performance than in 'neutral' mood), or to recommend an optimal schedule for the cognitive examination is at a 'normal' or neutral mood level.

Moreover, in the case of female subjects the menstrual cycle could be monitored and cognitive performance testing could be scheduled accordingly (or the performance value could be adjusted). For instance, it is shown that women performed better on mental rotation tasks around the follicular phase than around the ovulatory phase. In contrast, women's verbal fluency was significantly better around the ovulatory phase than around the follicular phase.

According to one aspect, biosignals with diurnal variations could be accessed to determine biological cycle data. For example, a biosignal of interest is the cortisol concentration which shows a diurnal pattern over the day with a peak in the morning at wake-up time (e.g. the cortisol awakening response), followed by a slow decrease over the day with a minimum cortisol level at night. The cortisol concentration can be obtained through blood sampling or unobtrusively through saliva samples. It should also be noted that cortisol levels reflects adrenal gland function and responds to acute and chronic stress. Accordingly, a cortisol level may be an indicator of an alertness of a subject, and thus their ability to perform cognitive tasks.

Thus, it may be best to perform the cognitive assessment when the cortisol level remains stable, with only limited diurnal variability. A preferred time could, for example, be around 2-5 pm for some subjects.

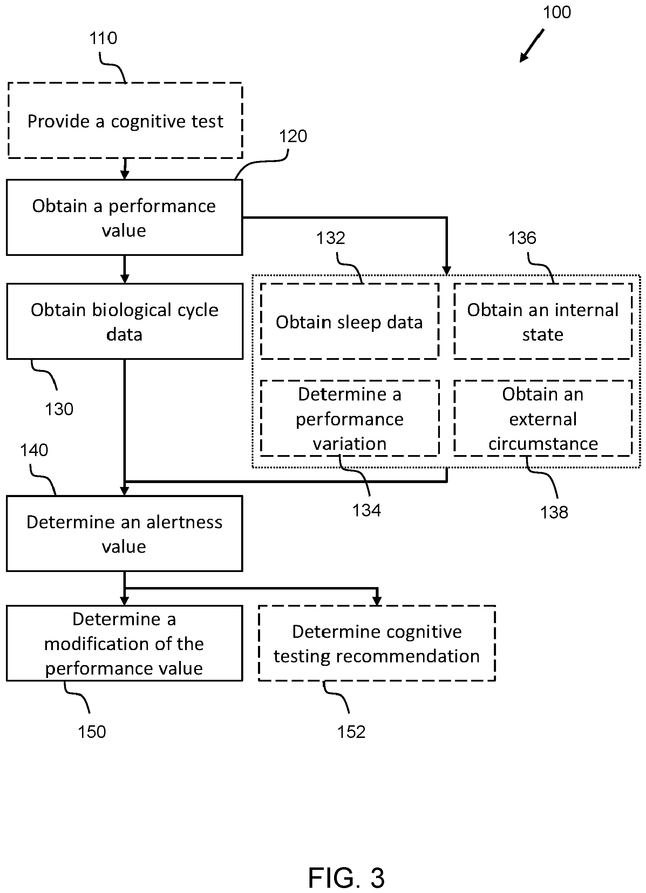

Fig. 3 presents a flow diagram of a method for assessing cognitive performance of a subject according to an embodiment of the invention. The method may aid and/or assist in the cognitive assessment of subjects (i.e. an individual or patient), such that an improved (i.e. more accurate) assessment of the cognitive abilities of the subject is facilitated. Indeed, such a method may be used alongside existing methods, as will be clear to the skilled person.

It should be understood that cognitive assessment/testing relates to the field of evaluating a cognitive/mental/neurological condition and/or ability of an individual. Such assessment is typically used for diagnosis of subjects, but also has uses in many other domains, such as in psychology and sociology.

Initially, step 110 may be performed, in which a cognitive test is provided to the subject, the cognitive test comprising a cognitive task.

Indeed, the cognitive test may prompt and/or encourage the user to perform one or more cognitive tasks suitable for cognitive assessment of the subject. The cognitive task may be suitable as a test for one or more different cognitive functions, such as short term and long term memory, reaction speeds and comprehension. Of course, the skilled person is well aware of a wide range of existing cognitive tasks suitable for assessing cognitive ability of a subject.

Providing the cognitive task may include simply presenting the cognitive task to the subject, or may comprise a number of steps to induce the subject to perform the cognitive task.

At step 120, a performance value is obtained, which describes the subject's performance in executing a cognitive task for cognitive assessment of the subject.

In other words, a suitable measure of the subject's ability in performing the task is obtained. For example, the performance value may relate to a time taken for the subject to complete the cognitive task. In other cases, the performance value may be an abstracted score (i.e. a percentage or grade) of the subject executing the cognitive task, which may be used to understand how well the subject performed the cognitive task. Of course, it should be understood that the performance value may comprise one or more pieces of information indicative of the subject's execution of the task.

The performance value may be obtained from a database or other memory storage, such that the method is performed retrospectively (i.e. after the cognitive task). Alternatively, the performance value may be obtained/acquired in real time to facilitate real time improvements to the cognitive assessment facilitated by embodiments of the invention. For example, the cognitive task may comprise interaction of the subject with an interface, which can automatically receive inputs, process said inputs and provide a performance value.

At step 130, biological cycle data is obtained, which describes parameters of at least one biological cycle of the subject.

Biological cycles/rhythms are any process dictated by the subject's internal body clock (and influenced by internal states and external circumstances), which have an impact on physiological states of the subject (i.e. hormone secretion, mood, etc.). Examples of such biological cycles include a circadian cycle, a menstrual cycle, a diurnal cycle, an ultradian cycle, an infradian cycle, and a circannual cycle of the subject. Indeed, these cycles and rhythms vary person-to-person, in that they may have a different frequency, different timings and a variety of impacts on the person.

Thus, the biological cycle data describes parameters useful for understanding the individual's biological cycle. As such, the biological cycle data may describe a rhythm (i.e. frequency, start point, etc.) of at least one of the biological cycles. For example, the biological cycle may be a menstrual cycle, which may have different start points (i.e. peaks on different days/times) and different lengths (i.e. an average number of days) between each subject.

The biological cycle may be obtained from the subject themselves (i.e. a questionnaire directly requesting such data, or asking for answers from which biological cycle data may be derived), from a care giver, or from a database of medical information of the subject. Of course, some biological cycles may be measured by hormone levels, etc.

At step 140, the biological cycle data is analyzed in order to determine an alertness value describing an alertness level of the subject during the cognitive task.

Once a biological cycle of a subject is understood, an alertness level of the subject may be determined/predicted/estimated at different times of day and/or on different days. By way of simple example, the circadian rhythm has a large impact on the sleepiness (and therefore alertness) of the subject as the day progresses. Thus, if the time (or scheduled time) of the cognitive task is known, then an associated alertness level of the subject can be known/derived.

Of course, many factors in conjunction with the biological cycle of the subject determine an alertness level of the subject. Obtaining such information may be useful for more accurately assessing the alertness level of the subject along with the biological cycle data. This is presented in (optional) steps 132-138, described below.

At (optional) step 132, sleep data is obtained, which is indicative of a quality of sleep of the subject prior to executing the cognitive task. In some cases, the sleep data of the subject comprises a start time of sleep, an end time of sleep, sleep stage data, and a perceived quality of sleep.

Accordingly, information/insights into the quality/sufficiency of recent sleep of the subject is acquired. As the (quality of) sleep of the subject is a large factor in the alertness of the subject, this may be key for accurately estimating/determining the alertness of the subject during the cognitive exam. Indeed, a quality of sleep can impact, and be impacted by, biological cycles, and thus a full understanding of sleep of the subject can aid in contextualizing the biological cycles of the subject.

The sleep data may be obtained from observations or directly from a questionnaire filled in by the subject. It is also becoming increasingly popular to wear sleep tracking devices, the data from which may be utilized to determine sleep quality.

When sleep data is obtained, determining the alertness value is further based on analyzing the sleep data.

Specifically, in some examples, analyzing the sleep data comprises processing the sleep data to determine a homeostatic pressure value describing sleep-pressure affecting the subject during the cognitive task. Thus, determining the alertness value is further based on analyzing the homeostatic pressure value.

Put another way, sleep data can be leveraged in order to determine a pressure on the subject to sleep as the day (i.e. an awake period) progresses. Such a pressure can be highly distracting and can heavily reduce alertness of the subject. Therefore, a full understanding of this can lead to an accurate assessment of the alertness level of the subject during execution of the cognitive task.

At (optional) step 134, a performance variation is determined. Indeed, in this case, the performance value may describe the subject's performance in executing a plurality of cognitive tasks (i.e. the performance value individually indicates the subject performance in performing a number of different tasks). Each of said plurality of cognitive tasks have a different task parameter describing a characteristic of the cognitive task.

In other words, the variation is performance of the subject performing a number of cognitive tasks is determined. From such a variation, an alertness of the subject may be derived. Indeed, it is known that a larger variation is performances (i.e. as a difficulty of the task increases) is indicative of a less alert subject, than a smaller variation of performance.

The task parameter is a characteristic of the task (i.e. a difficulty of action or interpretation). In this way the task parameter that varies between the tasks may comprise at least one of a stimulus intensity, a signal intensity, a memory load, a stimulus-response compatibility, and a time uncertainty.

In this case, determining the alertness value is further based on analyzing a variation in the subject's performance in executing each of the plurality of cognitive tasks.

Alternatively, or in addition, at step 134, the subject's performance in executing the cognitive task is compared to a reference performance value in order to determine the performance variation. Thus, determining the alertness value is further based on analyzing the comparison.

Differently to the variation above, the difference between the subject's performance compared to a reference (or benchmark) performance value (or values) can be determined. Accordingly, an alertness may be determined based on a difference between the obtained performance value and the reference performance value.

In one particular embodiment, the reference performance value is based on a historic measure of the subject's performance in executing a previous cognitive task. This may be useful when the subject is tested regularly, and therefore a variation from their own performance may be indicative of an alertness.

At (optional) step 136, an internal state is obtained, which describes a psychophysical condition of the subject during the cognitive task. The internal state comprises at least one of a mood, an emotional state, a physical condition, and a pharmacological substance intake.

At (optional) step 138, an external circumstance is obtained, which describes surroundings of the subject during the cognitive task. The external circumstance comprises at least one of an ambient temperate, an ambient noise level, an ambient light level, and a task constraint.

Indeed, internal states (i.e. a psychophysical condition/state/characteristic) as well as external circumstances/the surrounding environment may greatly impact the alertness of the subject. This may impact the biological cycle and/or sleep quality of the subject and thus their alertness.

As such, determining the alertness value may be further based on analyzing at least one of the internal state and the external circumstances of the subject.

At step 150, a modification to the performance value is determined based on the alertness level.

It is well documented how a subject's alertness (i.e. a sleepiness or arousal) impacts performance. Those who are not alert tend to perform noticeably worse than those who are alert. Understanding the difference between how the subject performed, and how they may have performed if they were alert (or in some cases, not alert) may be very useful for fully understanding the subject's (potential) performance, and thus an accurate assessment of their cognitive state/ability.

In some cases, the modification may be a quantifiable amount. In other cases, the modification may simply be an indication that the performance value is worse (or better) than it should be due to an alertness level of the subject. In this way, clinical decision making may be better informed.

In addition (or alternatively), at (optional) step 152, a cognitive testing recommendation is determined based on the determined alertness value. By having an awareness of the alertness of the subject during the test, a modification may be made to the test in real-time, or there may be other recommendations made (e.g. reduce an environmental noise, reduce a substance intake, and improve a sleep quality prior to a future examination.

In one exemplary embodiment, the cognitive testing recommendation may be indicative of a timing of another cognitive task for cognitive assessment of the subject. Indeed, sometimes the performance value may be far off that a modification provides inadequate information for the care giver to properly assess the cognitive ability of the subject. In this case, a recommendation as to the timing of the test would be beneficial, which is personalized to the subject so that it may be more likely that they are alert during the subsequent assessment session.

Of course, the modification and/or cognitive testing recommendation may then be provided to the subject and/or the care giver (i.e. a medical health professional) to properly understand their cognitive assessment. Alternatively, the modification may be applied automatically.

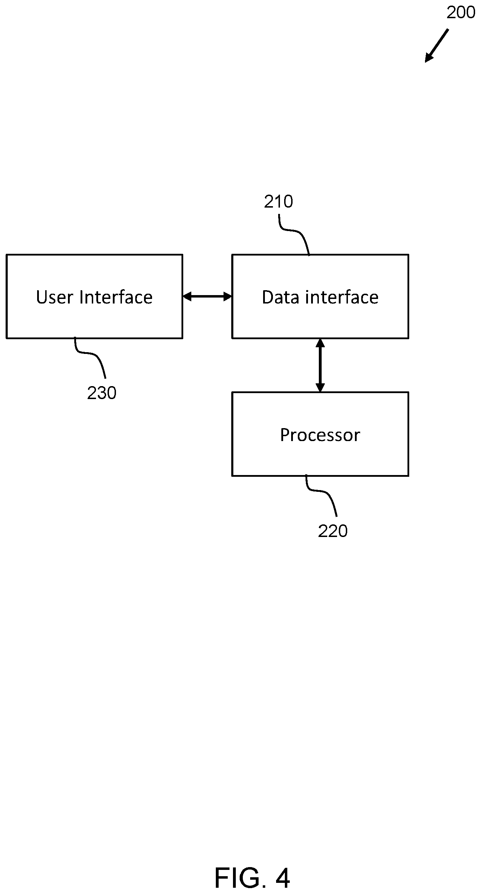

Moving on, Fig. 4 presents a simplified block diagram of a system 200 for assessing cognitive performance of a subject. The system comprises at least a data interface 210 and a processor 220. In some embodiments, there is further provided a user interface 230.

The data interface 210 is configured to obtain a performance value describing the subject's performance in executing a cognitive task for cognitive assessment of the subject. The data interface 210 also obtains biological cycle data describing parameters of at least one biological cycle of the subject.

In particular, the data interface 210 may be configured with the ability to access databases (i.e. medical records) and other information of the subject in order to obtain the performance value and biological cycle data. As explained above, the data interface 210 may also obtain sleep data, performance variations, internal states and external circumstances in a similar manner. In some cases, (i.e. when information is not available) the data interface 210 may request such information via the user interface 230, perhaps by the use of questionnaires.

The processor 220 is configured to analyze the biological cycle data to determine an alertness value describing an alertness level of the subject during the cognitive task. The processor 220 also determines a modification to the performance value based on the alertness level.

Accordingly, the processor 220 may have appropriate models and/or algorithms available for processing the data provided by the data interface 210. Specifically, the processor 220 is capable of using the biological cycle data to determine an awareness of the subject during execution of the cognitive task, and then using said data to provide a modification (and optionally a cognitive test recommendation) as described above.

Of course, this information may then be stored by the data interface 210. Alternatively (or in addition) the data interface 210 may then provide this information to a user interface 230 for communication to a user (i.e. the subject, and/or a care giver).

It should be appreciated that the processor 220, data interface 210 and user interface 230 may all be implemented on the same device, or may be distributed over multiple devices in a manner well understood by the skilled person.

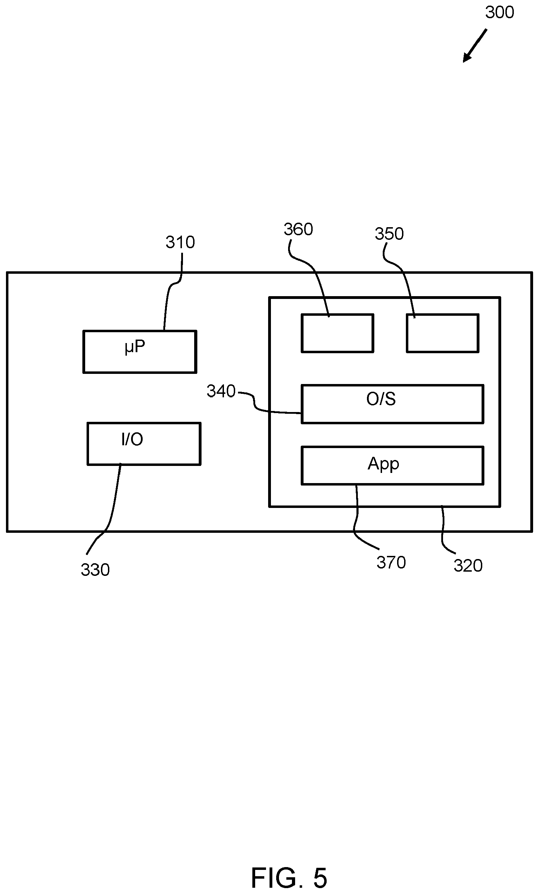

Fig. 5 illustrates an example of a computer 300 within which one or more parts of an embodiment may be employed. Various operations discussed above may utilize the capabilities of the computer 300. For example, one or more parts of a system for assessing cognitive performance of a subject may be incorporated in any element, module, application, and/or component discussed herein. In this regard, it is to be understood that system functional blocks can run on a single computer or may be distributed over several computers and locations (e.g. connected via internet).

The computer 300 includes, but is not limited to, PCs, workstations, laptops, PDAs, palm devices, servers, storages, and the like. Generally, in terms of hardware architecture, the computer 300 may include one or more processors 310, memory 320, and one or more I/O devices 370 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 310 is a hardware device for executing software that can be stored in the memory 320. The processor 310 can be virtually any custom made or commercially available processor, a central processing unit (CPU), a digital signal processor (DSP), or an auxiliary processor among several processors associated with the computer 300, and the processor 310 may be a semiconductor based microprocessor (in the form of a microchip) or a microprocessor.

The memory 320 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 320 may incorporate electronic, magnetic, optical, and/or other types of storage media. Note that the memory 320 can have a distributed architecture, where various components are situated remote from one another, but can be accessed by the processor 310.

The software in the memory 320 may include one or more separate programs, each of which comprises an ordered listing of executable instructions for implementing logical functions. The software in the memory 320 includes a suitable operating system (O/S) 350, compiler 340, source code 330, and one or more applications 360 in accordance with exemplary embodiments. As illustrated, the application 360 comprises numerous functional components for implementing the features and operations of the exemplary embodiments. The application 360 of the computer 300 may represent various applications, computational units, logic, functional units, processes, operations, virtual entities, and/or modules in accordance with exemplary embodiments, but the application 360 is not meant to be a limitation.

The operating system 350 controls the execution of other computer programs, and provides scheduling, input-output control, file and data management, memory management, and communication control and related services. It is contemplated by the inventors that the application 360 for implementing exemplary embodiments may be applicable on all commercially available operating systems.

Application 360 may be a source program, executable program (object code), script, or any other entity comprising a set of instructions to be performed. When a source program, then the program is usually translated via a compiler (such as the compiler 340), assembler, interpreter, or the like, which may or may not be included within the memory 320, so as to operate properly in connection with the O/S 350. Furthermore, the application 360 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 370 may include input devices such as, for example but not limited to, a mouse, keyboard, scanner, microphone, camera, etc. Furthermore, the I/O devices 370 may also include output devices, for example but not limited to a printer, display, etc. Finally, the I/O devices 370 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 370 also include components for communicating over various networks, such as the Internet or intranet.

If the computer 300 is a PC, workstation, intelligent device or the like, the software in the memory 320 may further include a basic input output system (BIOS) (omitted for simplicity). The BIOS is a set of essential software routines that initialize and test hardware at startup, start the O/S 350, and support the transfer of data among the hardware devices. The BIOS is stored in some type of read-only-memory, such as ROM, PROM, EPROM, EEPROM or the like, so that the BIOS can be executed when the computer 300 is activated.

When the computer 300 is in operation, the processor 310 is configured to execute software stored within the memory 320, to communicate data to and from the memory 320, and to generally control operations of the computer 300 pursuant to the software. The application 360 and the O/S 350 are read, in whole or in part, by the processor 310, perhaps buffered within the processor 310, and then executed.

When the application 360 is implemented in software it should be noted that the application 360 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 360 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The method of Fig. 3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figs illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figs. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for assessing cognitive performance of a subject, the method comprising:
obtaining (120) a performance value describing the subject's performance in executing a cognitive task for cognitive assessment of the subject;
obtaining (130) biological cycle data describing parameters of at least one biological cycle of the subject;
analyzing (140) the biological cycle data to determine an alertness value describing an alertness level of the subject during the cognitive task; and
determining (150) a modification to the performance value based on the alertness level.

2. The method of claim 1, further comprising obtaining (132) sleep data indicative of a quality of sleep of the subject prior to executing the cognitive task, and
wherein determining (140) the alertness value is further based on analyzing the sleep data.

3. The method of claim 2, wherein analyzing the sleep data comprises processing the sleep data to determine a homeostatic pressure value describing sleep-pressure affecting the subject during the cognitive task, and
wherein determining (140) the alertness value is further based on analyzing the homeostatic pressure value.

4. The method of claim 2 or 3, wherein the sleep data of the subject comprises a start time of sleep, an end time of sleep, sleep stage data, and a perceived quality of sleep.

5. The method of any of claims 1-4, further comprising determining (152) a cognitive testing recommendation based on the determined alertness value, the cognitive testing recommendation indicating timing of another cognitive task for cognitive assessment of the subject.

6. The method of any of claims 1-5, wherein the performance value describes the subject's performance in executing a plurality of cognitive tasks, wherein each of the plurality of cognitive tasks have a different task parameter describing a characteristic of the cognitive task, and
wherein determining (140) the alertness value is further based on analyzing a variation in the subject's performance in executing each of the plurality of cognitive tasks.

7. The method of claim 6, wherein the task parameter comprises at least one of a stimulus intensity, a signal intensity, a memory load, a stimulus-response compatibility, and a time uncertainty.

8. The method of any of claims 1-7, further comprising comparing (134) the subject's performance in executing the cognitive task with a reference performance value, and
wherein determining (140) the alertness value is further based on analyzing the comparison.

9. The method of claim 8, wherein the reference performance value is based on a historic measure of the subject's performance in executing a previous cognitive task.

10. The method of any of claims 1 to 9, wherein the biological cycle data of the subject describes at least one of a circadian cycle, a menstrual cycle, a diurnal cycle, an ultradian cycle, an infradian cycle, and a circannual cycle of the subject.

11. The method of any of claims 1-10, further comprising obtaining (136, 138) at least one of an internal state describing a psychophysical condition of the subject during the cognitive task, and an external circumstance describing surroundings of the subject during the cognitive task, and
wherein determining (140) the alertness value is further based on analyzing at least one of the internal state and the external circumstances of the subject.

12. The method of claim 11, wherein the internal state comprises at least one of a mood, an emotional state, a physical condition, and a pharmacological substance intake, and wherein the external circumstance comprises at least one of an ambient temperate, an ambient noise level, an ambient light level, and a task constraint.

13. The method of any of claims 1-12, further comprising providing (110) a cognitive test to the subject comprising the cognitive task.

14. A computer program comprising computer program code means adapted, when said computer program is run on a computer, to implement the method of any of claims 1 to 13.

15. A system (200) for assessing cognitive performance of a subject, the system comprising:
a data interface (210) configured to:
obtain a performance value describing the subject's performance in executing a cognitive task for cognitive assessment of the subject;
obtain biological cycle data describing parameters of at least one biological cycle of the subject; and
a processor (220) configured to:
analyze the biological cycle data to determine an alertness value describing an alertness level of the subject during the cognitive task; and
determine a modification to the performance value based on the alertness level.
